Europäisches Patentamt

(19) ))) European Patent Office

Office européen des brevets

(11) Publication number: **0 119 825**
**B1**

(12) **EUROPEAN PATENT SPECIFICATION**

(45) Date of publication of patent specification: **13.07.88**     (51) Int. Cl.⁴: **A 41 B 13/02**

(21) Application number: **84301717.9**

(22) Date of filing: **14.03.84**

(54) **Shaped disposable diapers with shaped elastically contractible waistband and method for their manufacture.**

(30) Priority: **18.03.83 US 476734**

(43) Date of publication of application:
**26.09.84 Bulletin 84/39**

(45) Publication of the grant of the patent:
**13.07.88 Bulletin 88/28**

(84) Designated Contracting States:
**AT BE CH DE FR IT LI LU NL SE**

(56) References cited:
**DE-A-3 016 197**
**US-A-3 881 489**
**US-A-3 912 565**
**US-A-4 324 245**
**US-A-4 341 216**
**US-A-4 352 355**

(73) Proprietor: **THE PROCTER & GAMBLE
COMPANY
301 East Sixth Street
Cincinnati Ohio 45201 (US)**

(72) Inventor: **Lash, Glen R.
5406 Bluebird Lane
Cincinnati Ohio 45239 (US)**

(74) Representative: **Gibson, Tony Nicholas et al
Procter & Gamble (NTC) Limited Whitley Road
Longbenton Newcastle upon Tyne NE12 9TS
(GB)**

Courier Press, Leamington Spa, England.

## Description

### Background of the invention
### Field of the invention

This invention concerns shaped disposable diapers, incontinent briefs, and the like having elastically contractible waistbands.

### Background art

Infants (and other incontinents) wear disposable diapers to receive and contain urine, feces, and other body fluids. Disposable diapers function both to contain the discharged materials and to isolate these materials from the body of the wearer and from the wearer's surroundings. Modern embodiments of disposable diapers frequently perform these tasks in a manner superior to that of traditional cloth diapers.

Disposable diapers normally comprise three elements: a liquid permeable topsheet designed to be placed next to the wearer's skin; a liquid impermeable backsheet which forms, in use, the outer surface of the diaper; and an absorbent element interposed between the topsheet and the backsheet.

The topsheet is frequently a hydrophobic non-woven fabric which is readily permeable to fluid. Its hydrophobicity tends to cause the surface in contact with the wearer's skin to be dry and the skin to be protected from fluids absorbed within the absorbent element.

The absorbent element receives and retains fluids which pass through the topsheet. It normally comprises a batt of airlaid wood pulp fibers.

The backsheet functions to contain fluids within the absorbent element thereby protecting ths wearer's outer garments and other surfaces from soiling by these fluids. Backsheets are commonly formed of fluid impermeable, vapor impermeable materials such as polyethylene film.

Disposable diapers having many different basic designs are known to the art. For example, Duncan and Baker in U.S. Patent Re 26,151, issued January 31, 1967, describe and claim a disposable diaper which has achieved wide acceptance and commercial success. Buell, in U.S. Patent 3,860,003, issued January 14, 1975, describes and claims another disposable diaper which, too, has achieved wide acceptance and commercial success. The diaper taught by Buell differs from that taught by Duncan and Baker in many respects, not the least of which is the provision in the Buell diaper of elasticized (or contractible) leg cuffs. Another embodiment of disposable diapers is described and claimed by Aziz and Blaney in European Patent Application EP—A—70584. The Aziz and Blaney diaper also provides elasticized (or contractible) leg cuffs, but is of a somewhat different design than that described by Buell.

Mesek et al in U.S. Patent 4,324,245, issued April 13, 1982; Pieniak et al in U.S. Patent 4,337,771, issued July 6, 1982; and Mesek et al in U.S. Patent 4,352,355, issued October 5, 1982 describe disposable diapers having elasticized cuffs and elasticized (or contractable) waistbands.

Strickland and Visscher in U.S. Patent 4,253,461, issued on March 3, 1981, describe and claim another form of disposable diaper sometimes referred to as an incontinent brief and intended to be worn by adults.

While the disposable diapers described above, particularly those described by Duncan and Baker, Buell, and Aziz and Blaney, function in exemplary manners, disposable diapers comprising fluid and vapor impermeable backsheets have sometimes been perceived as being somewhat hot and uncomfortable. Further, diapers provided with such impermeable backsheets are unable to self-dry as they otherwise would because evaporation of fluids from the absorbent element is precluded. To counteract this perception, and to permit self-drying, backsheets which are relatively impermeable to liquid but relatively permeable to vapor and which are known as breathable backsheets have been described. Breathable backsheets tend to provide a cooler garment and permit some measure of self-drying of the diaper while it is being worn. For example, Crowe, Jr. in U.S. Patent 3,156,242, issued on November 10, 1964, teaches the use of a microporous film as a breathable backsheet. Hartwell, in U.S. Patent 3,881,489, issued on May 6, 1975, teaches a breathable backsheet comprising, in combination, two layers: a low-void volume perforated thermoplastic film and a porous high-void volume hydrophobic tissue. Sisson, in U.S. Patent 3,989,867, issued on November 2, 1976, teaches a breathable backsheet provided with tapered hollowed bosses which prevent the passage of liquid while allowing vapor to pass readily therethrough. Obenour, in U.S. Patent 4,341,216, issued July 27, 1982, describes and claims a still different embodiment of a breathable backsheet.

Another improved diaper comprises an elastically contractible waistband which allows the diaper to breathe and which tends to resist waistband rollover. These benefits are achieved by providing disposable diapers with a waistband comprising an elastic element interposed between the topsheet and the backsheet and affixed to both the topsheet and the backsheet in such a way as to cause the formation of transverse channels between the topsheet and the elastic element and the backsheet and the elastic element. Transverse in this context means extending across ("transverse to") the waistband and that the channels extend from the outer diaper margin to an interior region of the diaper.

While the diapers described above are useful, developments providing more comfortable and serviceable diapers have still been sought.

### Summary of the invention

According to the present invention there is provided a shaped disposable diaper, comprising a liqiud permeable topsheet, a liquid impermeable backsheet, said backsheet being affixed to said topsheet, an absorbent element disposed between and of shorter length than said topsheet and said backsheet, and at least one waistband extending

2

from the laterally extending margin of the diaper to the laterally extending margin of said absorbent element, said waistband comprising an elongate elastomeric material in a heat-stable and elastic state, said elastomeric material being affixed to at least one of said topsheet and said backsheet; said at least one of said topsheet and said backsheet being gathered by reason of being affixed to said elastomeric material wherein the at least one waistband is folded into a C-fold configuration with fold regions at which said topsheet and said backsheet are folded; and wherein the at least one waistband is more gathered in the fold regions than in the regions outside the fold regions.

"Elastic" is used here to describe a material which can be elongated to a practical extent upon the application of tension and which will substantially return to its original configuration after the tension is released. In the context of the present invention, the heat stable state is dimensionally smaller than the heat unstable state. The elastomeric material is applied to the disposable diaper in its heat unstable state. The disposable diaper is folded into a preselected orientation, is restrained in that orientation, and is heated to such an extent as to cause the elastomeric material to assume its heat stable and elastic state. The resulting elastically contractable waistband assumes an essentially permanent set related to the folded configuration, which essentially permanent set tends to cause the disposable diaper to be shaped and to fit more closely about the trunk of the wearer. The resulting waistband also tends to resist waistband rollover.

Brief description of the drawings

Figure 1 is a perspective view of a prior art disposable diaper embodying an elastically contractible waistband and in a configuration as applied to an infant.

Figure 2 is a partially fragmented plan view of the disposable diapers shown in Figure 1 and in Figure 11, but in an unfolded configuration.

Figure 3 is an enlarged partial view of the waistband of the diaper of Figure 1 illustrating one preferred embodiment of an elastically contractible waistband.

Figure 4 is an enlarged partial view of the waistband of the diaper of Figure 1 illustrating another preferred embodiment of an elastically contractible waistband.

Figure 5 is an end view of the portion of the waistbands shown in Figures 3 and 4.

Figure 6 is a simplified schematic representation of an end view of the disposable diaper of Figure 10, which diaper has an elastomeric material in the waistband; the elastomeric material is shown in its heat unstable state.

Figure 7 is a simplified schematic end view of the disposable diaper shown in Figure 6 after the elastomeric material has assumed its heat stable and elastic state.

Figure 8 is a simplified schematic representation of the end of the disposable diaper of Figure 10, which diaper has elastomeric material in the waistband, which diaper has been folded into a C-fold configuration; the elastomeric material is shown in its heat unstable state.

Figure 9 is a simplified schematic representation of the disposable diaper shown in Figure 8 after the elastomeric material has assumed its heat stable and elastic state.

Figure 10 is a partially fragmented plan view of a simple disposable diaper.

Figure 11 is a perspective view of a diaper of this invention and is a configuration as applied to an infant.

Detailed description of the invention

While this specification concludes with claims particularly pointing out and distinctly claiming the subject matter regarded as the invention, it is believed that a better understanding of the invention can be achieved through careful reading of the following detailed description of the invention in conjunction with study of the attached drawings and the appended example.

Disposable diapers comprise three major elements: a topsheet; a backsheet; and an absorbent element. The topsheet forms the inside of the disposable diaper (i.e., that portion intended to be placed next to the wearer's skin). The backsheet generally forms the exterior surface of the disposable diaper. The absorbent element is interposed between the topsheet and the backsheet.

A disposable diaper is generally designed to be placed between and generally centered between the legs of an infant and secured about the infant by bringing the front portion of the diaper adjacent the front waist area of the infant and the rear portion of the diaper adjacent the rear waist area of the infant and securing the diaper in that position.

Optionally, disposable diapers comprise fastening tapes for securing the diaper about the infant. They also optionally comprise elastic members in the longitudinally extending margins to form an elastically contractible leg cuff or side flap. They also optionally comprise elastic elements in the laterally extending margins to form elastically contractible waistbands.

The waistband of a disposable diaper is that portion of the diaper which is intended to be placed adjacent the wearer's waist. While the waistband can comprise a separate element affixed to the body of the disposable diaper, it more often is an extension of other elements of the disposable diaper such as the backsheet or the topsheet or both the backsheet and the topsheet. Further, the waistband is generally considered to be that portion of the diaper extending from the laterally extending margin of the diaper to about the laterally extending margin of the absorbent element. Disposable diapers are normally constructed so as to have two waistbands: a front and a rear. While disposable diapers can be constructed so as to have a single unitary waistband encircling the waist of the wearer, such designs are not preferred. It is also possible to construct a disposable diaper having three or more waistband sections intended to be affixed about the waist of the wearer, but, these embodiments, too, are not preferred.

The present invention provides a shaped disposable diaper having at least one elastically contractible waistband. Further, the elastically contractible waistband comprises an elastomeric material having both a heat unstable state and a heat stable and elastic state. The elastomeric material is attached to the disposable diaper when the elastomeric material is in its heat unstable state; the elastomeric material is then heated under certain, defined conditions in such a manner that it assumes its heat stable but elastic state.

Massengale et al in U.S. Patent 3,819,401, issued June 25, 1974 and Koch et al in U.S. Patent 3,912,565, issued October 14, 1975, both teach a technique for preparing shirred, or gathered, elastic garments. In this technique, a heat contractible material is affixed to the garment when the heat contractible material is in its elongated and heat unstable state. The entire system is then heated thereby causing the heat contractible material to contract to its heat stable but elastic state thereby shirring, or gathering the garment.

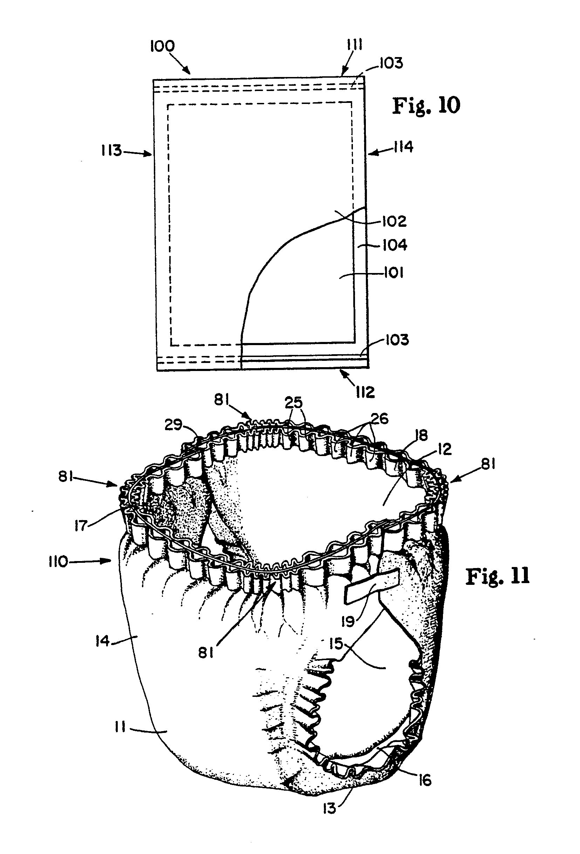

Any prior art disposable diaper, such as those taught by Duncan and Baker or by Buell or by Aziz and Blaney, comprising an absorbent core interposed between a topsheet and a backsheet, can incorporate the teachings of Massengale et al and Koch et al by having a strip of an elastomeric material (hereinafter defined in greater specificity) applied laterally across the width of the disposable diaper in one or both of its waist regions. Typically, the absorbent core does not extend fully to the lateral (waist) edges of the topsheet and backsheet which are typically coextensive. The elastomeric material is conveniently interposed between the topsheet and the backsheet in that region between the termination of the absorbent core and the termination of the essentially coextensive topsheet and backsheet. A simple disposable diaper is shown in partially cut away plan view in Figure 10.

In Figure 10, simple disposable diaper 100 comprises absorbent core 101 interposed between topsheet 102 and backsheet 104. Diaper 100 has longitudinal side margins 113 and 114. It also has lateral waist regions 111 (rear) and 112 (front). Strips of elastomeric material 103 are interposed between topsheet 102 and backsheet 104 and extend laterally across the diaper in that region between the termination of absorbent core 101 and the lateral edges of diaper 100. Elastomeric material strips 103 are affixed to both topsheet 102 and backsheet 104 by means not illustrated.

It must be noted that reference numerals are used consistently throughout all the figures and that the thickness of certain materials in the figures have been exaggerated for clarity.

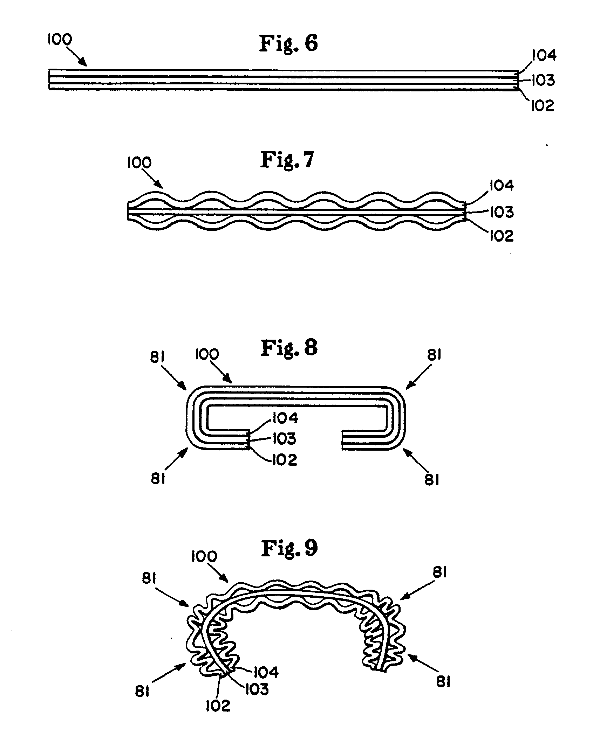

Figure 6 is an end view of diaper 100 as looking toward rear waist area 111. In this view, the thicknesses of the three elements have been exaggerated for clarity.

Figure 7 is the same view of diaper 100 as shown in Figure 6, but represents diaper 100 after elastomeric material strip 103 has been subjected to sufficient thermal energy to cause it to contract predominantly uniaxially. It can be readily seen that the contraction of elastomeric material strip 103 gathers, or shirrs, topsheet 102 and backsheet 104 more or less uniformly over their entire common lengths. This is what one obtains if one merely applies the teachings of Massengale et al or Koch et al to disposable diapers.

It has been surprisingly discovered that if the garment to which the elastomeric material is attached is a disposable diaper comprising an absorbent core, and if the diaper is folded while the elastomeric material is in its heat unstable form, the elastomeric material will contract nonuniformly when heated. Nonuniform contraction results in (1) nonuniform gathering of the materials attached to the elastomeric material; (2) a tendency of the diaper to retain a configuration analogous to its folded configuration; and (3) a decreased tendency to waistband rollover when the diaper is worn. The benefits flowing from a shaped configuration are better fit and decreased leakage around the waistband.

Figure 8 is the same end view of diaper 100 as shown in Figure 6 except that diaper 100 has been folded into a C-fold configuration and is held in that configuration by means not shown. Diaper 100 has four fold areas 81 as illustrated. The C-fold shown in this figure is an open C-fold. Closed folds where topsheet 102 actually contacts itself on the inner surfaces of the "C" are not only possible, but also are preferred in many situations.

Figure 9 is a view of diaper 100 analogous to that shown in Figure 8, except this view is taken after elastomeric material strip 103 has been subjected to sufficient thermal energy to cause it to substantially contract to its heat stable and elastic state. In Figure 9, the restraining means associated with, but not shown in, Figure 8, has been removed. It is to be noted that disposable diaper 100 retains a general "C" configuration, although not nearly so sharply defined as the C-fold configuration shown in Figure 8. Further, it is to be noted that neither topsheet 102 nor backsheet 104 is, uniformly gathered as shown in Figure 7. They are, in fact, more gathered, or shirred, in fold regions 81 than in regions outside fold regions 81.

While expressing an intention not to be bound by the following comments, it can be theorized that the benefits of the present invention flow from a tendency of the absorbent core to resist the contraction of the elastomeric material in those regions where the absorbant core is intact. The bending of the absorbant core, as at the folds, tends to decrease the amount of resistance the core engenders thereby allowing the elastomeric material to contract more fully in the regions associated with the folds.

The elastomeric materials useful in the present invention include a number of materials well known to those skilled in the art. For example, the polyurethane described in the hereinbefore mentioned patent to Koch et al and the plasticized vinyl chloride described in the hereinbefore mentioned patent to Massengale et al can be used. Further, the compositions comprising a mixture of a thermoplastic resin material (or other organic, normally solid heat flowable material) in an elastomeric material as described by Cook in U.S. Patent Re 28,688, can also be used.

A preferred elastomeric material useful herein is a blend of ethylene propylene rubber with ethylene vinyl acetate which has been extruded into a film as made by Exxon Chemical Company of Florham Park, New Jersey, and which has been tentered. During tentering, the film is placed in an oven at approximately 65°C and, after softening, it is stretched to approximately four times its original length in one direction. The stretched film is then allowed to cool. The polymers are now predominantly uniaxially oriented and the film essentially retains its new dimension and is in a heat unstable state. (It should be noted that this particular material is still elastic even in its heat unstable state.) Subsequent treatment of the film as with heated air at 68°C is sufficient to cause the film to reassume its heat stable and elastic state.

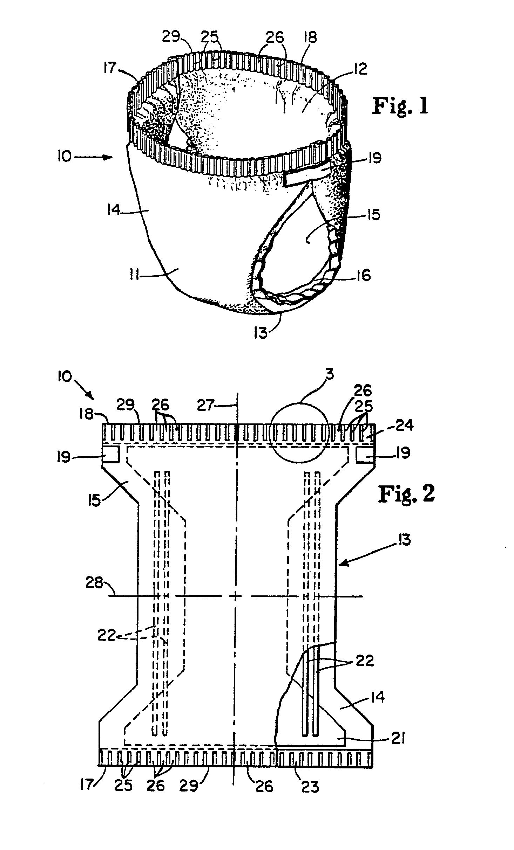

A diaper embodiment in which the present invention finds preferred utility is illustrated in Figure 1 through 5.

Figure 1 is a perspective view of a prior art disposable diaper, but one in which the present invention is preferred for use. The diaper illustrated in Figure 1 is based on the disposable diaper design taught in the hereinbefore mentioned patent to Buell. (While this is a preferred diaper design for use in the present invention, it must be realized that the present invention can be used, and is contemplated for use, with other disposable diaper designs. Several such designs are described in the hereinbefore mentioned patents and European patent application; other designs can be readily envisioned by those skilled in the art.)

Referring now to Figure 1, disposable diaper 10 is shown in perspective in a configuration as if it were applied about an infant. Disposable diaper 10 comprises a front portion 11 and a rear portion 12 with a crotch portion 13 interposed therebetween. In use, crotch portion 13 is placed between the legs of the infant and front portion 11 and rear portion 12 are placed, respectively, along the front and rear lower portions of the wearer's trunk. Topsheet 15 forms the inner surface of disposable diaper 10 while backsheet 14 forms its outer surface. Side flaps (or leg cuffs) 16 fit about the wearer's thighs. In use, front waistband 17 and rear waistband 18 are placed adjacent the wearer's waist regions on, respectively, the front and rear portions of the wearer's trunk. Disposable diaper 10 is held in position about the wearer by fastening tape 19. Outer margin of waistband 29 is shown in Figure 1 as the upper edge of disposable diaper 10. Transverse regions of securement 25 and transverse regions of nonsecurement 26 in the waistbands are discussed more fully hereinafter.

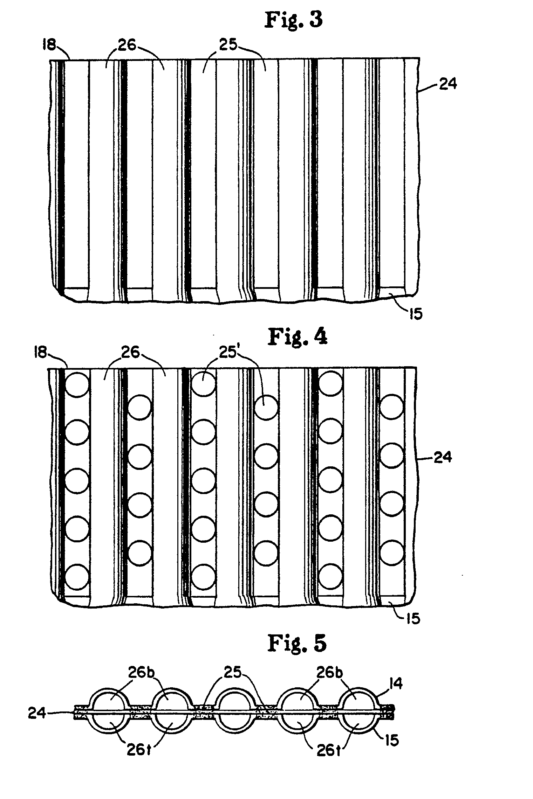

Figure 2 is a partially cut away plan view of disposable diaper 10 opened out into a planar configuration. Topsheet 15 is, in this illustration, the upper surface of the diaper while backsheet 14 is the lower surface. Absorbent element 21 is interposed between topsheet 15 and backsheet 14.

As illustrated, disposable diaper 10 is generally symmetrical about longitudinal center line 27 and lateral center line 28. While this is a preferred configuration, it is not necessary that disposable diaper 10 be symmetrical. An asymmetric orientation about lateral center line 28, as when crotch portion 13 is transposed toward front waistband 17, is quite useful.

Disposable diaper 10 is provided with elastic members 22 in the side margins thereof running generally parallel to longitudinal center line 27. In the embodiment illustrated, two elastic members 22 are placed on either side of disposable diaper 10; single or multiple elastic members can be used. The embodiment illustrated is, however, preferred.

Fastening tapes 19 are secured to disposable diaper 10 adjacent rear waistband 18.

Front waist elasteromic element 23 and rear waist elastomeric element 24 are positioned, respectively, in front waistband 17 and rear waistband 18 adjacent outer margin of waistband 29. In the embodiment illustrated in Figures 1 and 2, disposable diaper 10 comprises elastic waist elements in both the front and the rear waistbands. While this is a preferred embodiment, the present invention is useful in diaper designs having only the front or only the rear waistband elasticized.

Transverse regions of securement 25 and transverse regions of nonsecurement 26 are also illustrated in Figure 2.

One major function of backsheet 14 is to prevent body fluids from escaping from disposable diaper 10 and soiling the wearer's outer garments and other surfaces in contact with the disposable diaper. Any compliant, non-irritating planar material which is impermeable to body fluids can be used as backsheet 14. Suitable materials are described with particularity in the hereinbefore mentioned patents and patent application. A preferred backsheet is formed from polyethylene film having a thickness of from about 0.012 to about 0.051 millimeter (mm).

Breathable backsheets (i.e., backsheets that permit the passage of vapor and air while retarding the passage of liquid) useful in the present invention are described in the hereinbefore mentioned patents to Crow, Jr., Hartwell, Sisson, and Obenour.

The size of backsheet 14 is dictated by the exact diaper design selected and the size of the infant intended to be the wearer; it can be readily ascertained by those skilled in the art.

Topsheet 15 can be any compliant, soft feeling, non-irritating (to the wearer's skin) planar material. It functions to contact the wearer's skin, to receive fluid discharges, to allow the discharges to pass readily therethrough into the absorbent element, and to isolate the wearer's skin from the fluids in the absorbent element. To aid in effective performance of the last function, the topsheet is preferably hydrophobic.

Topsheet 15 can be porous paper made from natural or synthetic fibers or mixtures thereof, non-woven fabric made from natural or synthetic fibers or mixtures thereof, apertured plastic film, porous foam, or the like. Examples of suitable topsheets are described in the hereinbefore mentioned patents and patent application.

A preferred topsheet is spun bonded non-woven polyester fabric made from fibers of from 0.24 to 0.275 tex, having a basis weight of 17 grams (g) per square meter ($M^2$). Another preferred topsheet material has a basis weight of 22 g per $M^2$ and comprises 65% (by weight) staple length, 0.16 tex polyester fibers (such as Kodel type 411 polyester fibers as sold by Tennessee Eastman Corporation, Kingsport, Tennessee);

15% crimped, staple length, 0.16 tex rayon fibers; and

20% acrylic copolymer binder (such as Celanese CPE 8335 as sold by Celanese Corporation of Charlotte, North Carolina), "Staple length" refers to fibers having a length of at least 15 mm.

Still another preferred topsheet is constructed from polypropylene fibers which have been carded and thermally bonded in a spaced-apart pattern. Fibers 3.8 centimeters (cm) long and of from 0.16 to 0.33 tex are suitable. A preferred topsheet of this type has a basis weight of 24 g per $M^2$.

Suitable topsheets can also be constructed from apertured plastic films such as those described by Radal and Thompson in U.S. Patent 4,342,314, issued August 3, 1982; Ferguson and Landrigan in U.S. Patent 4,341,217, issued July 27, 1982; and Thompson in U.S. Patent 3,929,135, issued December 30, 1975.

As with the case of backsheet 14, the size of topsheet 15 is dictated by the exact diaper design selected.

Absorbent element 21 can be any means which is generally compressible, conformable, non-irritating to the wearer's skin, and which is capable of absorbing and retaining fluid.

Absorbent element 21 can be constructed from any of a variety of materials commonly used in disposable absorbent articles and which are described in the hereinbefore incorporated patents. Examples of suitable absorbent materials include creped cellulose wadding, absorbent foams, absorbent sponges, super absorbent polymers, and, preferably, comminuted and airlaid wood pulp fibers commonly referred to as absorbent fluff. An absorbent fluff having a density of from 0.05 to 0.175 g per $cm^3$ is generally acceptable.

As in the case of backsheet 14 and topsheet 15, the size of absorbent element 21 is dictated by the exact diaper design selected.

Optionally, absorbent element 21 can have associated with either or both planar faces envelope tissues (not illustrated in the drawings) comprising any permeable material well known to those skilled in the art, such as wet strength tissue paper. When used, envelope tissues are generally coextensive with absorbent element 21 and either coterminus therewith or folded up and about the laterally extending margins thereof. Envelope tissues can optionally be secured to absorbent core 21 by any means well known to those skilled in the art.

Absorbent element 21 is interposed between backsheet 14 and topsheet 15. The diaper design selected determines whether or not the three elements are coterminus although, in general, either backsheet 14 or topsheet 15 or both extend beyond the margins of absorbent element 21. In the present invention, both backsheet 14 and topsheet 15 preferably extend beyond the laterally extending margins of absorbent element 21 and are essentially coterminus along their laterally extending margins.

Optionally, backsheet 14 can be secured to absorbent element 21 by any convenient means (not illustrated in the drawings) well known to those skilled in the art. Examples of suitable means are parallel beads of adhesive (such as hot melt adhesive) and double sided adhesive tape; each extend essentially the entire longitudinal length of absorbent element 21.

Elastic members 22 serve to contract or gather the cuffs (longitudinally extending margins) of disposable diaper 10 and maintain them in contact with the legs of the wearer thereby providing improved fit and reducing fluid leakage from the diaper. One material which can be used for elastic elements 22 is an elastic tape having a cross section of 0.18 mm by from 1.5 mm to 6.4 mm and made from natural rubber as available from East Hampton Rubber Company of Stuart, Virginia, under the trademark L-1900 Rubber Compound. Other suitable elastic members can be made from natural rubber elastic tapes sold under the trademarks Fulflex 9211 and Fulflex 9111 by Fulflex Company, of Scotland, North Carolina.

The length of elastic elements 22 is dictated by the precise diaper design chosen. In the design illustrated in Figures 1 and 2, elastic elements 22 extend a major portion of the longitudinal length of disposable diaper 10, but terminate outside the waist regions of disposable diaper 10.

Elastic members 22 are operably associated with disposable diaper 10 by securing them to the diaper adjacent its longitudinally extending margins by elastic attachment means which are not shown in the Figures. The elastic attachment means should be flexible and of sufficient adhesiveness to hold elastic members 22 in their stretched condition substantially indefinitely. One suitable means is hot melt adhesive. A more detailed description of the manner in which elastic members 22 should be positioned and secured to disposable diaper 10 is given in the hereinbefore incoporated patent to Buell.

Elastic members 22 are affixed to disposable diaper 10 in an elastically contractible condition so that in a normally unrestrained configuration elastic members 22 effectively contract or gather the diaper material adjacent elastic members 22. Elastic members 22 can be affixed to disposable diaper 10 in an elastically contractible condition in at least two ways. For example, elastic members 22 can be stretched to an elongated orientation and affixed to disposable diaper 10 while disposable diaper 10 is in an uncontracted condition. Alternatively, disposable diaper 10 can be contracted (in crotch portion 13, for example by pleating) and elastic members 22 can be affixed to the contracted disposable diaper 10 while the elastic members are in their relaxed or unstretched orientation.

Front waist elastomeric element 23 and rear waist elastomeric element are each formed of elastomeric material as hereinbefore described. Waist elastomeric elements 23 and 24 are at least 0.6 cm wide, preferably at least 1.6 cm wide. While the maximum width of waist elastomeric elements 23 and 24 is determined by the diaper design and matters of economy, they generally are no wider than 3.8 cm.

In the embodiment illustrated in Figures 1 and 2 waist elastomeric elements 23 and 24 each extend across essentially the entire lateral width of disposable diaper 10. While this is a preferred construction, the present invention is useful in designs wherein waist elements 23 and 24 extend across only a portion of the lateral width of the diaper. Waist elastomeric elements 23 and 24 should, however, extend across a major portion of the lateral width of disposable diaper 10.

The topsheet, backsheet, and each waist elastomeric element are affixed together by transverse regions of securement while the waist elastomeric elements are in their heat unstable state and all three elements are essentially fully, but not elastically, extended. The system is later heated (as with heated air) and the waist elastomeric element is allowed to return to its heat stable and elastic state.

Transverse regions of securement 25 are shown in a generalized representation in Figures 1 and 2. More specific embodiments of transverse regions of securement 25 are depicted in Figures 3 and 4 which are enlarged views of a portion of rear waistband 18 indicated by reference numeral 3 in Figure 2. In these views, the elastomeric material is in its heat stable and elastic state.

In this discussion of Figures 3 and 4, reference shall be made to rear waistband 18 and the components thereof. The same comments can be made about front waistband 17 and its components.

Transverse regions of securement 25 extend essentially across the whole width of waist elastomeric element 24.

The term "transverse" as used in this context refers to an orientation generally perpendicular to the major laterally extending dimension of waistband 18. That is to say, since rear waistband 18 extends laterally across the width of disposable diaper 10 and is generally parallel to lateral center line 28, the transverse regions of securement 25 extend across rear waistband 18 in an orientation essentially parallel to longitudinal center line 27; they are directed generally from outer margin of waistband 29 to the center of disposable diaper 10. As illustrated, transverse regions of securement 25 are shown to be at essentially right angles to lateral center line 28 and to the lateral extent of waistband 18. This is the preferred orientation. One can, however, depart from true transversity without destroying the benefits conferred. The departure from true (or absolute) transversity becomes too great when channels (as hereinafter discussed) are no longer formed extending essentially across the width of waistband 18. In general, departure from transversity becomes too great for practical operation of the present invention when the departure from transversity exceeds 45° from true transversity (or perpendicularity to lateral center line 28).

The term "essentially across" is used in this context to indicate that transverse regions of securement 25 need not extend absolutely across the entire width of waist elastomeric element 24 so long as they extend sufficiently far across the width thereof to provide the channels discussed hereinafter.

In Figure 3, transverse regions of securement 25 are shown as essentially regularly spaced unitary zones of sealing attaching waist elastomeric element 24 to topsheet 15 and backsheet 14 which is not visible in Figure 3 or 4. The precise means for providing the zones of sealing can be readily selected by those skilled in the art. Examples include adhesive attachment, solvent sealing and the like. Preferably, ultrasonic welding is used.

As illustrated in Figures 3, 4 and 5, the points of attachment of both topsheet 15 and backsheet 14 to waist elastomeric element 24 are in register (i.e. are coextensive). This is a preferred orientation, but the points of attachment of topsheet 15 to waist elastomeric element 24 can be offset from the adjacent points of attachment of backsheet 14 to waist elastomeric element 24. In such a situation there will be offset transverse regions of securement on either side of the waist elastomeric element.

Figure 4 illustrates an alternate embodiment of transverse regions of securement 25'. In this embodiment, the transverse regions of securement comprise discrete spaced zones of sealing, preferably ultrasonic welds, effectively attaching the materials together and forming the channels hereinafter described. Preferably the discrete spaced zones are circular or elliptical.

Transverse regions of securement 25 can be from 0.15 to 1.0 cm wide (i.e. in the dimension generally parallel to lateral centerline 28. They are preferably regularly spaced, but they can be nonuniformly spaced. They are preferably from 0.3 to 1.5 cm apart as measured from center to center.

Figure 5 illustrates the functioning of the transverse regions of securement. Figure 5 is an end view of the portions of rear waistband 18 shown in Figure 3 and Figure 4 with rear waist elsatomeric element 24 in its heat stable and elastic state. In Figure 5, transverse regions of securement 25 are shown as darkened portions for emphasis. Topsheet 15 and backsheet 14 are shown gathered. These gathers constitute and define transverse regions of nonsecurement 26b between backsheet 14 and rear waist elastomeric element 24 and transverse regions of nonsecurement 26t between topsheet 15 and rear waist elastomeric element 24. These transverse regions of nonsecurement 26b and 26t form open gathers or channels from the margin of the diaper extending to the interior of the diaper and terminating in the region adjacent the laterally extending edges of absorbent element 21. These open channels allow the diaper to breathe by allowing the exchange of air and vapor between the interior of the diaper and the surrounding atmosphere even when the diaper is secured about an infant.

At the same time as transverse regions of nonsecurement 26b and 26t are formed, topsheet 15 and backsheet 14 form structures in the nature of corrugations. These corrugations extend transversely across the width of rear waistband 18 tend to stiffen the waistband thereby tending to prevent waistband rollover (i.e., the bending of the waistband about itself).

As noted hereinbefore, the diaper illustrated in Figure 1 does not embody the teachings of the present invention. That is to say, when the diaper was heated to cause the waist elastomeric elements to change from their heat unstable state to their heat stable and elastic state, the diaper was in an unrestrained, generally planer configuration. As illustrated in Figure 1, relatively uniform corrugations and channels were produced. A diaper 110 made as the diaper of Figure 1, but treated according to the teachings of this invention, is shown in Figure 11. Before heating the diaper to cause the waist elastomeric elements to change from their heat unstable state to their heat stable and elastic state, the front and rear waist regions 17 and 18 were folded into a closed C-fold configuration as indicated in Figure 8. The diaper was restrained in this configuration during the heating. Following the heating operation, which converted the waist elastomeric elements to their heat stable and elastic state, and after the restraining means were removed, front waistband 17, rear waistband 18, and diaper 110 exhibited generally shaped configurations even in the absence of securement with adhesive tapes 19. The size of the corrugations in channels was not uniform with the corrugations and channels being smaller in regions of fold 81. Thus, Figure 11 illustrates a diaper of the present invention.

As indicated hereinbefore, the diaper embodiments described in some detail are preferred embodiments. Other diaper designs embodying elastically contractible waistbands can benefit from the teachings of this invention.

In the foregoing discussion, the elastomeric material which was affixed to the diaper in an unstable state and was then treated to cause it to be transfromed into a stable and elastic state has been discussed in terms of a "heat unstable" elastomer. More broadly, the elastomeric materials useful herein are those materials that have an unstable state relative to some other stable and elastic state and which can be caused to be transformed from the unstable to the stable state by the application of any form of energy or by any other convenient treatment. The most convenient and the most practical form of energy is heat and the materials are described in such terms herein. The form of energy can be any other similar form of energy such as ultraviolet, infrared, microwave, or gamma radiation.

Still further, the present invention has been described in terms of an elastomeric material which is affixed to the diaper in its unstable state and is later transformed to its stable and elastic state. While, in general, the stable state is an absolute state, it is not necessary that it be, it is only required that the state following treatment be relatively more stable than the state preceeding treatment and that the state following treatment be sufficiently stable for practical use. It is, of course, necessary that the material be elastic in its stable state.

Example

Diapers according to the present invention are constructed following the basic design described in the aforementioned patent to Buell. This diaper design provides for two (front and rear) waistbands.

The absorbent element comprises absorbent fluff having a density of 0.09 g per cm$^3$ and a basis weight of 1100 g per M$^2$ in the crotch portion and 350 g per M$^2$ near the waist portions. It is generally hourglass-shaped and is 38.7 cm long, 25.4 cm wide at each of its laterally extending margins, and 9.6 cm wide in the crotch portion. It is symmetrical about its longitudinal center line, but asymmetrical about its lateral center line in that the crotch portion is centered 21.6 cm from the rear lateral margin.

The topsheet comprises the thermally bonded polypropylene material hereinbefore mentioned and the backsheet 0.04 mm thick polyethylene film. Both also are hourglass-shaped and are 43.8 cm long and 30.5 cm wide at their laterally extending margins.

During construction, the absorbent element is interposed between the topsheet and the backsheet which are essentially coextensive and coterminus. Hot-melt adhesive glue beads running parallel to the longitudinal center line secure the backsheet to the absorbent element. They also secure the backsheet to the topsheet in the cuff regions.

The cuff portions of the diapers are elasticized by incorporating therein two elastic members in each longitudinally extending margin of the diaper at the crotch portion. Each is made of Fulflex 9211 and is 2.4 mm wide and 0.18 mm thick; their relaxed length is 19.6 cm. They are extended to 220% of their original lengths at the time of attachment. These elastic members are centered about the crotch portion. The pattern defined by the pair of members is centered 9.4 cm from the longitudinal center line of the diaper and parallel thereto. The two elastic members in each diaper margin are centered on parallel lines 1.6 cm apart.

Each diaper waistband extends the lateral width of the diaper and is 30.5 cm long; each waistband is 2.5 cm wide in the transverse direction. The waist elastomeric element used in each waistband is the hereinbefore described ethylene propylene rubber blended with ethylene vinyl acetate which is tentered as described. Each is 30.5 cm long. 2.5 cm wide, and 0.04 mm thick and is secured to both the topsheet and the backsheet. At the time of securement the topsheet and the backsheet are in fully extended configurations.

Transverse regions of securement comprising discrete zones of ultrasonic welds are used to affix each waist elastomeric element to both the topsheet and to the backsheet. The tranverse regions of securement each comprise seven discrete elliptical zones of ultrasonic welds each having a major axis of 1.9 mm and a minor axis of 1.0 mm; each individual ellipse is set with its major axis at an angle of 45° to the transverse direction. The transverse regions of securement are generally regularly spaced along each waistband and their centers are 6.4 mm apart.

Adhesive fastening tapes are adhesively affixed to the diaper.

Each assembled diaper is folded in a closed C-fold as described above and several are restrained in that configuration. The folded diaper is heated with air at 68°C so that the elastomeric material shrinks to its heat stable and elastic state. The diapers of this invention exhibit different degrees of gathering along the waist band when the restraining force is removed and the diapers are allowed to assume a loose "C" configuration.

When worn by infants, these diapers perform in a satisfactory manner and the waistbands thereof tend to conform more effectively about infants' waists than do similar diapers which are subjected to heat treatment while unrestrained.

**Claims**

1. A shaped disposable diaper (10, 100, 110) comprising a liquid permeable topsheet (15, 102), a liquid impermeable backsheet (14, 104), said backsheet being affixed to said topsheet, an absorbent element (21, 101) disposed between and of shorter length than said topsheet (15, 102) and said backsheet (14, 104), and at least one waistband (17, 18, 29) extending from the laterally extending margin of the diaper to the laterally extending margin of said absorbent element, said waistband comprising an elongate elastomeric material (23, 24, 103) in a heat stable and elastic state, said elastomeric material being affixed to at least one of said topsheet (15, 102) and said backsheet (14, 104); said at least one of said topsheet and said backsheet being gathered by reason of being affixed to said elastomeric material characterised in that the at least one waistband (17, 18, 29) is folded into a C-fold configuration with fold regions (81) at which said topsheet (15, 102) and said backsheet (14, 104) are folded; and in that the at least one waistband (17, 18, 29) is more gathered in the fold regions (81) than the regions outside of the fold regions (81).

2. A shaped disposable diaper according to claim 1 wherein said elastomeric material (23, 24, 103) is affixed to at least one of said topsheet (15, 102) and said backsheet (14, 104) by transverse regions of securement (25) defining therebetween transverse regions of nonsecurement (26, 26b, 26t) extending from the outer margin of said waistband (17, 18, 29) across essentially the entire width of said elastomeric material.

3. A shaped disposable diaper according to claim 2 wherein said transverse regions of securement (25) comprise zones of sealing which may be unitary or spaced apart.

4. A shaped disposable diaper according to claim 3 wherein said zones of sealing comprise ultrasonic welds.

5. A method of making a shaped disposable diaper comprising the steps of:
   1) forming the diaper in the fully extended flat condition by assembling a topsheet, a backsheet, an absorbent element disposed between said topsheet and said backsheet and at least one waistband disposed between, and secured to at least one of, said backsheet and said topsheet so as to extend across at least a major portion of the width of the diaper, said waistband comprising an elongate elastomeric material having a heat unstable state and a heat stable and elastic state, said elastomeric material being in its heat unstable state;
   2) forming said flat diaper into a c-fold configuration about fold lines extending longitudinally of the diaper, said c-fold intersecting said elastomeric material;
   3) restraining said diaper in said c-fold configuration;
   4) heating said elastomeric material to such an extent as to cause it to change from its heat unstable state by contraction to its heat stable and elastic state thereby causing the backsheet and/or topsheet secured thereto to become gathered in a non uniform manner; and
   5) cooling said elastomeric material thereby to leave it with a permanent set in the c-fold configuration.

6. A method according to claim 5 wherein said elastomeric material is affixed to at least one of said topsheet and said backsheet by transverse regions of securement defining therebetween transverse regions of nonsecurement extending from the outer margin of said waistband across essentially the entire width of said elastomeric material.

7. A method according to claim 6 wherein said transverse regions of securement comprise zones of sealing which may be unitary or spaced apart.

8. A method according to claim 7 wherein said zones of sealing comprise ultrasonic welds.

**Patentansprüche**

1. Geformte Wegwerfwindel (10, 100, 110), die eine flüssigkeitsdurchlassige Abdecklage (15, 102), eine flüssigkeitsundurchlassige Unterlage (14, 104), wobei die genannte Unterlage an der genannten Abdecklage befestigt ist, ein absorbierendes Element (21, 101), das zwischen der genannten Abdecklage (15, 102) und der genannten Unterlage (14, 104), angeordnet ist und von kürzerer Längenerstreckung als die genannte Abdecklage und die genannte Unterlage ist, und wenigstens ein Taillenband (17, 18, 29) umfaßt, welches sich von dem sich in seitlicher Richtung erstreckenden Rand der Windel bis zu dem sich in seitlicher Richtung erstreckenden Rand des genannten absorbierenden Elementes erstreckt, wobei das genannte Taillenband ein langgestrecktes elastomeres Material (23, 24, 103) in einem wärmestabilen und elastischen Zustand umfaßt, und wobei das genannte elastomere Material an wenigstens einer der aus der genannten Abdecklage (15, 102) und aus der genannten Unterlage (14, 104) gebildeten Komponenten befestigt ist; und wobei wenigstens eine der genannten, aus der genannten Abdecklage und der genannten Unterlage gebildeten Komponenten aufgrund des Umstandes gerafft ist, daß sie an dem genannten elastomeren Material befestigt ist, dadurch gekennzeichnet, daß das wenigstens eine Teillenband (17, 18, 29) in einer C-förmigen Faltungskonfiguration mit Faltungsbereichen (81) gefaltet ist, in denen die genannte Abdecklage (15, 102) un die genannte Unterlage (14, 104) gefaltet sind; und daß das wenigstens eine Teillenband (17, 18, 29) in den Faltungsbereichen (81) stärker gerafft ist als in den außerhalb der Faltungsbereiche (81) gelegenen Bereichen.

2. Geformte Wegwerfwindel nach Anspruch 1, worin das genannte elastomere Material (23, 24, 103) an wenigstens einer der aus der genannten Abdecklage (15, 102) und aus der genannten Unterlage (14, 104) gebildeten Komponenten durch quer-verlaufende Befestigungsbereiche (25) befestigt ist, welche dazwischen quer-verlaufende Nichtbefestigungsbereiche (26, 26b, 26t) begrenzen, welche letzteren sich vom äußeren Rand des genannten Taillenbandes (17, 18, 29) quer über im wesentlichen die gesamte Breite des genannten elastomeren Materials erstrecken.

3. Geformte Wegwerfwindel nach Anspruch 2, wobei die genannten quer-verlaufenden, Befestigungsbereiche (25) Versiegelungszonen darstellen, welche einheitlich oder im Abstand voneinander angeordnet sein können.

4. Geformte Wegwerfwindel nach Anspruch 3, wobei die genannten Versiegelungszonen Ultraschallschweißungen darstellen.

5. Verfahren zur Herstellung einer geformten Wegwerfwindel, welches die folgenden Stufen umfaßt:

(1) das Fertigen der Windel im voll ausgestreckten, flachen Zustand, durch Zusammenfügen einer Abdecklage, einer Unterlage, eines zwischen der genannten Abdecklage und der genannten Unterlage angeordneten, absorbierenden Elementes, und wenigstens eines Teillenbandes, welches letztere zwischen der genannten Unterlage und der genannten Abdecklage derart angeordnet ist, und an wenigstens einer der aus der genannten Unterlage und der genannten Abdecklage gebildeten Komponenten derart befestigt ist, daß es sich quer über wenigstens einen Hauptanteil der Breite der Windel erstreckt, wobei das genannte Teillenband ein langgestrecktes elastomeres Material umfaßt, welches einen wärmeinstabilen Zustand sowie einen wärmestabilen und elastischen Zustand aufweist, und wobei sich das genannte elastomere Material in seinem wärmeinstabilen Zustand befindet;

(2) das Verformen der genannten flachen Windel zu einer C-förmigen Faltungskonfiguration durch Faltung um Faltungslinien herum, die sich in der Längsrichtung der Windel erstrecken, wobei die genannte C-Faltung das genannte elastomere Material schneidet;

(3) das Beschranken der genannten Windel in der genannten C-förmigen Faltungskonfiguration;

(4) das Erhitzen des genannten elastomeren Materials in einem solchen Ausmaß, daß dadurch eine Umwandlung des genannten elastomeren Materials von seinem wärmeinstabilen Zustand durch Zusammenziehung auf seinen wärmestabilen und elastischen Zustand bewirkt wird, und wobei weiterhin bewirkt wird, daß die an dem genannten elastomeren Material befestigte Unterlage und/oder Abdecklage auf nicht gleichförmige Art und Weise gerafft wird/werden; und

(5) das Abkühlen des genannten elastomeren Materials, wodurch dasselbe mit einer permanenten Anordnung in der C-förmigen Faltungskonfiguration zurückbleibt.

6. Verfahren nach Anspruch 5, wobei das genannte elastomere Material an wenigstens einer der aus der genannten Abdecklage und aus der genannten Unterlage gebildeten Komponenten durch quer-verlaufende Befestigungsbereiche befestigt ist, welche dazwischen quer-verlaufende Nichtbefestigungsbereiche begrenzen, welche letzteren sich vom äußeren Rand des genannten Taillenbandes quer über im wesentlichen die gesamte Breite des genannten elastomeren Materials erstrecken.

7. Verfahren nach Anspruch 6, wobei die genannten quer-verlaufenden Befestigungsbereiche Versiegelungszonen darstellen, welche einheitlich oder im Abstand voneinander angeordnet sein können.

8. Verfahren nach Anspruch 7, wobei die genannten Versiegelungszonen Ultraschallschweißungen darstellen.

## Revendications

1. Une couche à jeter après usage (10, 100, 110) comprenant une feuille supérieure perméable aux liquides (15, 102), une feuille-support imperméable aux liquides (14, 104), ladite feuille-support étant fixée à ladite feuille supérieure, un élément absorbent (21, 101) disposé entre et plus court que ladite feuille supérieure (15, 102) et ladite feuille-support (14, 104), et au moins une bande de ceinture (17, 18, 29), s'étendant de la lisière s'étendant latéralement de la couche à la lisière s'étendant latéralement dudit élément absorbant, ladite bande de ceinture comprenant une matière élastomère allongée (23, 24, 103) dans une condition résistant à la chaleur et élastique, ladite matière élastomère étant fixée à au moins une desdites feuilles supérieure (15, 102) et audit support (14, 104); au moins une desdites feuilles supérieure et feuille support étant froncée pour être fixée à ladite matière élastomère, caractérisée en ce qu'au moins une bande de ceinture (17, 18, 29) est plissée sous forme de plis en C avec des zones de plis (81) au niveau desquelles ladite feuille supérieure (15, 102) et ladite feuille-support (14, 104) sont plissées; et en ce qu'au moins une bande de ceinture (17, 18, 29) est plus froncée dans les zones de plis (81) que dans les zones extérieures aux zones de plis (81).

2. Une couche à jeter après usage conformée suivant la revendication 1, dans laquelle ladite matière élastomère (23, 24, 103) est fixée à au moins une desdites feuilles supérieure (15, 102) et feuille-support (14, 104) par des zones transversales de fixation (25) définissant entre elles des zones transversales de non-fixation (26, 26b, 26t) s'étendant de la lisière extérieure de ladite bande de ceinture (17, 18, 29) à travers essentiellement toute la largeur de ladite matière élastomère.

3. Une couche à jeter après usage conformée selon la revendication 2, dans laquelle lesdites zones transversales de fixation (25) comportent des zones de scellage étanches qui peuvent être unitaires ou espacées.

4. Une couche à jeter après usage conformée selon la revendication 3 dans laquelle lesdites zones de scellage étanches comprennent des soudures ultrasoniques.

5. Un procédé de fabrication d'une couche à jeter après usage conformée comprenant les étapes de:

   (1) mise en forme de la couche dans la condition plane totalement étendue en assemblant une feuille supérieure, une feuille-support, un élément absorbant disposé entre ladite feuille supérieure et ladite feuille-support et au moins une bande de ceinture disposée entre elles, et fixée à au moins une desdites feuilles-support et supérieure de manière à s'étendre à travers au moins une partie majeure de la largeur de la couche, ladite bande de ceinture comprenant une matière élastomère allongée ayant une condition instable à la chaleur et une condition résistant à la chaleur et élastique, ladite matière élastomère étant dans sa condition instable à la chaleur;

   (2) mise en forme de ladite couche plate suivant une configuration de plis en C autour de lignes de pliage s'étendant longitudinalement à la couche, lesdits plis en C croisant ladite matière élastomère;

   (3) maintien de ladite couche dans ladite configuration de plis en C;

   (4) chauffage de ladite matière élastomère de manière suffisante pour l'amener à venir par contraction de son état instable à la chaleur à sa condition résistant à la chaleur et élastique pour faire en sorte que la feuille-support et/ou la feuille supérieure fixées à celle-ci soient froncées d'une manière non-uniforme; et

   (5) refroidissement de ladite matière élastomère de manière à ce qu'elle garde un état permanent en forme de plis en C.

6. Procédé selon la revendication 5, dans lequel ladite matière élastomère est fixée à au moins une desdites feuilles, supérieure et feuille-support dans des zones transversales de fixation définissant entre elles des zones transversales de non-fixation s'étendant de la lisière extérieure de ladite bande de ceinture à travers essentiellement la totalité de la largeur de ladite matière élastomère.

7. Un procédé selon la revendication 6, dans lequel lesdites zones transversales de fixation comportent des zones de scellage qui peuvent être unitaires ou espacées.

8. Un procédé selon la revendication 7, dans lequel lesdites zones de scellage comprennent des soudures ultrasoniques.

Fig. 1

Fig. 2

0 119 825

## Fig. 3

## Fig. 4

## Fig. 5

**Fig. 6**

**Fig. 7**

**Fig. 8**

**Fig. 9**

Fig. 10

Fig. 11